# FASCICULE DE BREVET EUROPEEN

(11) **EP 1 487 406 B1**
(45) Date de publication et mention de la délivrance du brevet: **11.08.2010**
(21) Numéro de dépôt: 03738199.3
(22) Date de dépôt: 26.03.2003
(51) Int. Cl.: A61K 9/00, A61K 47/34

(54) **COMPOSITION THERMOREVERSIBLE DESTINEE AU TRAITEMENT DES HYPOSIALIES ET ASIALIES**
HITZE-REVERSIBLE ZUSAMMENSETZUNG ZUR BEHANDLUNG VON HYPOSALIE UND ASIALIE
HEAT-REVERSIBLE COMPOSITION TREATING HYPOSALIA AND ASIALIA

(30) Priorité: 26.03.2002 FR 0203728
(43) Date de publication de la demande: 22.12.2004
(73) Titulaire: Ellipse Pharmaceuticals, 33600 Pessac (FR)
(72) Inventeur: AUZERIE, Jack, F-33560 Sainte-Eulalie (FR)
(74) Mandataire: Fantin, Laurent
(86) Numéro de dépôt international: PCT/FR2003/000949
(87) Numéro de publication internationale: WO 2003/080020

(56) Documents cités:
- WO-A-02/07691
- WO-A-02/41837
- WO-A-96/34608
- FR-A- 2 751 534
- US-A- 5 496 541

## Description

La présente invention couvre une composition pharmaceutique de salive artificielle, permettant de compenser les insuffisances de sécrétion salivaire dites xérostomies qu'il s'agisse de diminutions de la sécrétion salivaire appelées hyposialies ou d'absences totales de sécrétion dénommées asialies.

La xérostomie se traduit par une sécheresse de la bouche. Le patient ressent à l'intérieur de la bouche et sur la langue des brûlures, des picotements, la bouche semble pâteuse et collante. Les patients souffrant de ce trouble ont des difficultés à avaler et à manger des aliments secs notamment. Dans certains cas, la simple prise de comprimés ou gélules est difficile.

Les aliments semblent fades, sans goût et même l'élocution est difficile.

Sur le long terme, la xérostomie peut provoquer des lésions du type ulcération au niveau de la muqueuse buccale, de la langue et des lèvres.

De plus, l'absence de salive a des conséquences sur l'hygiène buccodentaire et on constate chez les patients une augmentation du nombre de caries, l'apparition de mycoses buccales particulièrement difficiles à éradiquer.

L'absence de salive provoque des difficultés de sommeil avec pour conséquence directe une fatigue physique.

Les causes de la xérostomie sont nombreuses et notamment d'origine médicamenteuse.

On peut citer de nombreuses familles de médicaments très diverses mais parmi les plus utilisés, on peut citer les anorexiques, les anxiolytiques, les anticholinergiques et antispasmodiques, les anticonvulsants, les antidépresseurs, les antidiarréhiques, les antiémétiques, les antihistaminiques, les antihypertenseurs, certains analgésiques et anti-inflammatoires, les antiparkinsoniens, les antipsychotiques, les broncho-dilatateurs, les décongestionnants, les diurétiques, les sédatifs et les relaxants musculaires.

La maladie de Sjörgen, maladie auto-immune, forme d'arthrite qui frappe les femmes après la ménopause diminue la sécrétion des glandes lacrymales mais aussi salivaires.

Dans certains cas, les malades infectés par le virus HIV et plus particulièrement chez les enfants, le gonflement des glandes salivaires provoquent des xérostomies.

On peut citer d'autres affections dont les effets provoquent des xérostomies : l'arthrite rhumatoïde, le lupus érythémateux, le diabète, l'hypertension, les désordres endocriniens, ou les dysfonctionnements de la thyroïde.

Dans certains cas de cancer, suivant la localisation, les radiothérapies mises en oeuvre pour les éradiquer peuvent détruire les glandes salivaires de façon irréversible, par fibrose des glandes salivaires. Il peut y avoir des épisodes précoces qui succèdent aux séances de radiothérapies mais la xérostomie n'est alors que passagère.

La composition selon la présente invention a pour objet de pallier les conséquences liées à la xérostomie sachant que les traitements connus ne donnent pas satisfaction.

L'art antérieur propose des médicaments qui agissent par voie systémique. La pilocarpine est une substance active à effet cholinergique qui est utilisée, elle stimule les muscles disposés autour des glandes salivaires pour augmenter la production de salive. La cevimeline stimule aussi la production de salive.

Néanmoins, les effets secondaires sont importants car ces principes agissent également en d'autres points et conduisent à l'apparition de suées, à des désordres gastro-intestinaux, de l'hypotension, des rhinites, des troubles visuels.

Les contre-indications sont les patients souffrant d'asthme, de glaucome et les femmes enceintes.

De plus, lorsque les glandes salivaires sont totalement détruites, la stimulation ne génère pas d'effets tangibles.

D'autres produits agissent localement pour stimuler la salivation dans la sphère buccale comme les bonbons sans sucre, les pâtes à mâcher ou des formulations à base de maltose.

L'efficacité est temporaire et ces produits ne sont pas adaptés en cas d'atteinte grave des glandes salivaires.

Des produits sont proposés comme substitut de la salive comme des bains de bouche à base de chlorhéxidine ou d'héxamédine. De tels produits n'ont qu'une faible rémanence nécessitant des applications répétées.

La demande de brevet européen EP 788 368 décrit des préparations aqueuses de polymères. Le produit commercial est dénommé Xialine, sous forme de spray. Néanmoins, il est difficile d'obtenir une imprégnation régulière de la muqueuse buccale à cause de la viscosité de la préparation qui est quasiment constante quelle que soit la température.

On connaît aussi par la demande de brevet WO96 34608, une composition ayant une fonction de lubrification, notamment dans le cas des xérostomies.

Cette demande présente une composition qui inclut un polymère β-glucane de façon à lui conférer un comportement rhéologique proche de celui de la salive. Il est en effet recherché une viscosité sensiblement identique à celle de la salive. On ne dispose pas là d'une composition très fluide à température ambiante en sorte de faciliter l'application dont la viscosité augmente pour se gélifier et permettre une certaine adhérence sur la paroi buccale pour prolonger son action.

Le brevet US 5 496 541 décrit un produit dentaire qui utilise un système surfactant ternaire incluant un poloxamère combiné avec des polysaccharides et de la cellulose. Cette composition permet d'obtenir une composition qui adhère bien à la muqueuse et aux dents.

Un tel produit ne présente pas une viscosité qui varie dans le sens recherché, à savoir faible viscosité à température ambiante et augmentation de la viscosité à la température buccale voire atteinte du point de gélification.

La présente invention propose une composition utilisable comme substitut de la salive, sous forme thermoréversible.

Cette forme permet de compenser les inconvénients des compositions connues et notamment conduit à une meilleure imprégnation de la cavité buccale du fait de la forme liquide et faiblement visqueuse lors de l'application. La gélification permet ensuite une bonne adhésivité sur la muqueuse buccale.

D'autres avantages apparaissent et notamment la possibilité de conditionner la composition en flacons pressurisés, sous forme stérile. Ceci est bénéfique pour les patients qui présentent des lésions des muqueuses.

La composition selon la présente invention est maintenant décrite en détail selon différents modes de réalisation.

La composition selon l'invention comprend comme premier composé un polymère thermoréversible choisi parmi les copolymères synthétiques formés d'oxyde d'éthylène et d'oxyde de propylène appelés Poloxamères notamment le Poloxamère 407, commercialisé sous le nom de Lutrol F 127.

Ce polymère permet de conserver le produit à température ambiante sous forme liquide tandis qu'il devient beaucoup plus visqueux à température du corps humain.

Un deuxième composé associé au premier est un polymère bioadhésif choisi parmi la famille des polyvinylpyrrolidone, le carboxyméthylcellulose et ses sels, l'hydroxypropylméthylcellulose, l'hydroxypropylcellulose, les alginates, les dérivés de la gomme arabique adragante et karaya, l'acide alginique et ses sels, les polymères des acides acryliques, méthacryliques et leurs dérivés.

De façon non nécessaire mais souhaitable, notamment pour éviter les contaminations microbiennes, on peut ajouter des conservateurs comme les esters de l'acide parahydroxybenzoïque, l'acide sorbique, l'acide benzoïque, les sels d'ammonium quaternaire comme le chlorure de benzalkonium ou la chlorhéxidine.

En complément, on peut ajouter à cette composition des substances anti-oxydantes afin de stabiliser les caractéristiques du produit, notamment pour éviter les phénomènes d'oxydation des dérivés des poloxamères.

Comme antioxydants, on peut citer les tocophérols, les dérivés de la vitamine E, le palmitate d'ascorbyle, la vitamine C, les flavonoïdes d'origine naturelle, les isoflavonoïdes ou les polyphénols.

Afin de se rapprocher des caractéristiques de la salive, on peut introduire des enzymes présentes dans la salive et qui participent à la digestion et à l'hygiène buccodentaire, telles que les alpha-amylases, le lysozyme et la lactoferrine. Afin d'illustrer la composition selon l'invention des exemples sont maintenant donnés à titre non limitatif mais simplement illustratif.

### Exemple 1 :

Les quantités sont exprimées en % en poids.

| | |
|---|---|
| Poloxamère 407 | : 15 % |
| Hydroxypropylcellulose | : 1 % |
| (Klucel EF) | |
| Eau purifiée | : Quantité Suffisante Pour 100 % |

Pour réaliser cette composition, le mode opératoire consiste à dissoudre dans l'eau purifiée à température ambiante le poloxamère 407 à l'aide d'une défloculeuse et à ajouter progressivement l'hydroxypropylcellulose.

La salive artificielle est placée sous agitation à 4°C pendant quelques heures pour assurer un débullage.

### Exemple 2 :

| | |
|---|---|
| Poloxamère 407 | : 15 % |
| Hydroxypropylcellulose | : 0,5 % |
| (Natrosol 250) | |
| Eau purifiée | : Quantité Suffisante Pour 100 % |

Pour réaliser cette composition, on dissout dans l'eau purifiée sous agitation à température ambiante, l'hydroxypropylcellulose.

On ajoute ensuite le polymère thermoréversible progressivement à la défloculeuse.

La salive artificielle est placée sous agitation à 4°C pendant quelques heures pour assurer un débullage.

### Exemple 3 :

| | |
|---|---|
| Poloxamère 407 | : 15 % |
| Gomme karaya | : 0,5 % |
| Eau purifiée | : Quantité Suffisante Pour 100 % |

Pour réaliser cette composition, on dissout la gomme karaya dans l'eau purifiée sous agitation à température ambiante.

On ajoute ensuite le polymère thermoréversible progressivement à la défloculeuse.

La salive artificielle est placée sous agitation à 4°C pendant quelques heures pour assurer un débullage.

### Exemple 4 :

| | |
|---|---|
| Poloxamère 407 | : 15% |
| Hydroxypropylméthylcellulose | : 2 % |
| ( Pharmacoat 606) | |
| Eau purifiée | : Quantité Suffisante Pour 100 % |

On dissout simultanément l'hydroxypropylméthylcellulose et le polymère dans l'eau purifiée sous agitation à température ambiante.

La salive artificielle est placée sous agitation à 4°C pendant quelques heures pour assurer un débullage.

### Exemple 5 :

| | |
|---|---|
| Poloxamère 407 | :15 % |
| Carbomères | : 0,5 % |
| (Carbopol 5984) | |
| Eau purifiée | : Quantité Suffisante Pour 100 % |

On dissout d'abord le carbomère dans l'eau purifiée sous agitation à température ambiante.

Le poloxamère est ajouté progressivement à la défloculeuse.

La salive artificielle est placée sous agitation à 4 °C pendant quelques heures pour assurer un débullage.

### Exemple 6 :

| | |
|---|---|
| Poloxamère 407 | : 15 % |
| Hydroxpropylméthylcellulose: | : 2 % |
| (Pharmacoat 606) | |
| Vitamine E | : 2% |
| Eau purifiée | : Quantité Suffisante Pour 100 % |

On dissout le poloxamère et l'hydroxypropylméthylcellulose simultanément dans l'eau purifiée sous agitation à température ambiante avec introduction de la vitamine E

La salive artificielle est placée sous agitation à 4°C pendant quelques heures pour assurer un débullage.

Afin de montrer l'effet de synergie obtenu avec la composition selon la présente invention, on mesure la viscosité à l'aide d'un appareillage du commerce.

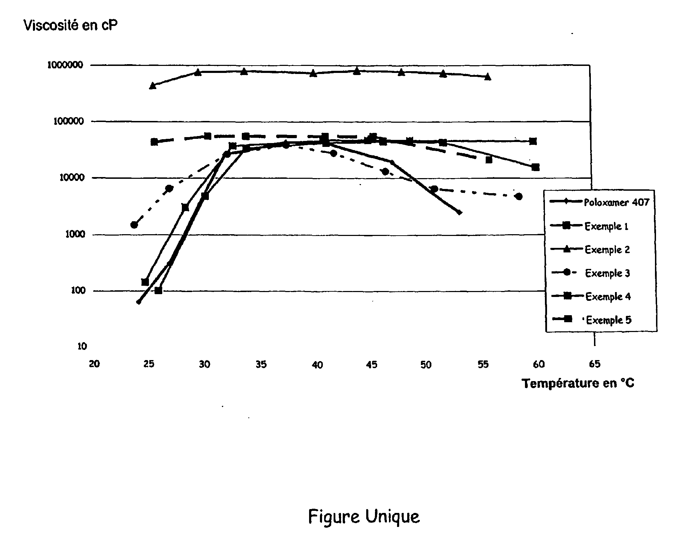

On constate sur la figure unique annexée que pour les compositions des exemples 1 à 5, les viscosités présentent toutes un maximum de viscosité entre 35 et 40°C, ce qui correspond au seuil de gélification dans la zone recherchée.

La viscosité est faible à température ambiante et augmente à la température buccale. Le composé polymère à viscosité croissante présente une viscosité optimale à la température buccale atteignant un point de gélification.

Cette composition comprend en combinaison un polymère bioadhésif avec le composé polymère à viscosité croissante. Ce polymère est introduit dans la composition à raison de 1% à 30% en poids et le polymère bioadhésif est introduit dans ladite composition à raison de 0,1% à 10,0% en poids.

A température ambiante, la composition est liquide, avec une faible viscosité, ce qui permet son conditionnement en flacons pulvérisateurs, pressurisés, en capsules molles, en tubes souples ou en conditionnements unitaires stériles.

## Revendications

1. Composition thermoréversible destinée à compenser les insuffisances salivaires, **caractérisée en ce qu'**elle comprend au moins un composé polymère dont la viscosité est faible à température ambiante et qui augmente à la température buccale de façon à atteindre une viscosité optimale à la température buccale jusqu'à un point de gélification.

2. Composition thermoréversible selon la revendication 1, **caractérisée en ce qu'**elle comprend un polymère bioadhésif en combinaison avec le composé polymère à viscosité croissante.

3. Composition thermoréversible selon la revendication 2, **caractérisée en ce que** le polymère bioadhésif est choisi parmi la famille des polyvinylpyrrolidone, le carboxyméthylcellulose et ses sels, l'hydroxypropylméthylcellulose, l'hydroxypropylcellulose, les alginates, les dérivés de la gomme arabique adragante et karaya, l'acide alginique et ses sels, les polymères des acides acryliques, méthacryliques et leurs dérivés.

4. Composition thermoréversible selon l'une quelconque des revendications précédentes, **caractérisée en ce qu'**elle comprend un antioxydant.

5. Composition thermoréversible selon l'une quelconque des revendications précédentes, **caractérisée en ce qu'**elle comprend au moins une enzyme telle que les alpha-amylases, le lysozyme et la lactoferrine.

6. Composition thermoréversible selon l'une quelconque des revendications précédentes, **caractérisée en ce que** le polymère à viscosité croissante est introduit dans ladite composition à raison de 1% à 30% en poids et le polymère bioadhésif est introduit dans ladite composition à raison de 0,1 % à 10,0 % en poids.

## Claims

1. Thermoreversible composition to compensate salivary insufficiency, **characterized in that** it comprises at least one polymeric compound whose viscosity is low at ambient temperature and which increases at the buccal temperature so as to reach an optimum viscosity at the buccal temperature up to the point of gelification.

2. Thermoreversible composition according to claim 1, **characterized in that** it comprises a bioadhesive polymer in combination with the polymeric compound of increasing viscosity.

3. Thermoreversible composition according to claim 2, **characterized in that** the bioadhesive polymer is selected from the family of polyvinylpyrrolidone, carboxymethylcellulose and its salts, hydroxypropylmethylcellulose, hydroxypropylcellulose, alginates, derivatives of adragante and karaya gum arabic, alginic acid and its salts, polymers of acrylic acids, methacrylates and their derivatives.

4. Thermoreversible composition according to any one of the preceding claims, **characterized in that** it comprises an antioxidant.

5. Thermoreversible composition according to any one of the preceding claims, **characterized in that** it comprises at least one enzyme such as alpha-amylases, lysozyme and lactoferrine.

6. Thermoreversible composition according to any one of the preceding claims, **characterized in that** the polymer with increasing viscosity is introduced into said composition in the amount of 1 to 30% by weight and the bioadhesive polymer is introduced into said composition in the amount of 0.1% to 10.0% by weight.

## Patentansprüche

1. Thermoreversible Zusammensetzung, die dazu bestimmt ist, Speichelinsuffizienz zu kompensieren, **dadurch gekennzeichnet, dass** sie wenigstens eine Polymerverbindung umfasst, deren Viskosität bei Raumtemperatur gering ist und bei Mundtemperatur zunimmt, so dass eine optimale Viskosität bei Mundtemperatur bis zu einem Gelpunkt erreicht wird.

2. Thermoreversible Zusammensetzung nach Anspruch 1, **dadurch gekennzeichnet, dass** sie ein bioadhäsives Polymer in Kombination mit der Polymerverbindung mit zunehmender Viskosität umfasst.

3. Thermoreversible Zusammensetzung nach Anspruch 2, **dadurch gekennzeichnet, dass** das bioadhäsive Polymer aus der Familie der Polyvinylpyrrolidone, Carboxymethylcellulose und ihren Salzen, Hydroxypropylmethylcellulose, Hydroxypropylcellulose, Alginaten, Derivaten von Gummi arabicum Traganth und Karaya, Alginsäure und ihren Salzen, Polymeren der Acryl-, Methacrylsäure und ihren Derivaten ausgewählt ist.

4. Thermoreversible Zusammensetzung nach einem der vorhergehende Ansprüche, **dadurch gekennzeichnet, dass** sie ein Antioxidans umfasst.

5. Thermoreversible Zusammensetzung nach einem der vorhergehenden Ansprüche, **dadurch gekennzeichnet, dass** sie wenigstens ein Enzym, wie Alpha-Amylasen, Lysozym und Lactoferrin umfasst.

6. Thermoreversible Zusammensetzung nach einem der vorergehenden Anspruch, **dadurch gekennzeichnet, dass** das Polymer mit zunehmender Viskosität in einem Verhältnis von 1 bis 30 Gew.-% und das bioadhäsive Polymer in einem Verhältnis zwischen 0,1 und 10,0 Gew.-% in die Zusammensetzung eingebracht ist.
